# EUROPÄISCHE PATENTSCHRIFT

(11) **EP 2 906 261 B1**
(45) Veröffentlichungstag und Bekanntmachung des Hinweises auf die Patenterteilung: **13.12.2017**
(21) Anmeldenummer: 13779519.1
(22) Anmeldetag: 08.10.2013
(51) Int. Cl.: A61L 2/07, A61L 2/20

(54) **STERILISATIONSVERFAHREN**
STERILISATION METHOD
PROCÉDÉ DE STÉRILISATION

(30) Priorität: 11.10.2012 DE 102012019937; 11.10.2012 US 201261712485 P
(43) Veröffentlichungstag der Anmeldung: 19.08.2015
(73) Patentinhaber: Fresenius Medical Care Deutschland GmbH, 61352 Bad Homburg (DE)
(72) Erfinder: KREBER, Stefan, 66113 Saarbrücken (DE)
(74) Vertreter: Rau, Jenspeter
(86) Internationale Anmeldenummer: PCT/EP2013/003013
(87) Internationale Veröffentlichungsnummer: WO 2014/056601

(56) Entgegenhaltungen:
- EP-A1- 1 484 069
- WO-A1-01/76646
- AU-B2- 526 378
- S.S. BLOCK: "Disinfection, Sterilization, and Preservation", 31. Dezember 2001 (2001-12-31), XP002717908, Seite 706-710, 761, Seiten 706-710 Seite 761

## Beschreibung

### Zusammenfassung

Die Erfindung betrifft das Gebiet der Sterilisation mit dem fraktionierten Vakuum-Dampfverfahren.

### Stand der Technik

Das fraktionierte Vakuumdampf-Verfahren gehört zu den vom Robert Koch-Institut anerkannten Desinfektionsverfahren und findet zumeist Anwendung bei schwer entlüftbaren Produkten, wie z. B. Schlauch- oder Kassettensystemen. Ein solches Verfahren ist z.B. beschrieben in Wallhäußer, Praxis der Sterilisation, Desinfektion, Antiseptik und Konservierung, in S.S. Block: "Disinfection, Sterilization and Preservation und der DE 101 14 758 A1.

### Aufgabenstellung

Einwegartikel für medizinische Anwendungen müssen oft nach der Produktion sterilisiert werden. Handelt es sich um Kunststoffprodukte mit luftgefüllten Hohlräumen, wie z.B. die oben erwähnten Schlauch- oder Kassettensysteme, die nicht für eine trockene Heißsterilisation geeignet sind, wird meist das fraktionierte Vakuumdampf-Verfahren angewendet.

Bei diesem Verfahren werden die zu sterilisierenden Produkte in eine vakuumdicht abzuschließende Kammer eingelegt. Anschließend wird durch mehrmaliges Evakuieren der Kammer im Wechsel mit Einströmen von Sterilisationsmedium die Luft in der Kammer und in den zu sterilisierenden Produkten durch Sterilisationsmedium ersetzt. Ist die gesamte Atmosphäre in der Sterilisationskammer mit Sterilisationsmedium gesättigt, erfolgt eine Inkubationsphase mit dem Sterilisationsmedium, bis die gewünschte Entkeimung erreicht ist. Nach dieser Inkubationsphase wird dann das Sterilisationsmedium durch den Wechsel zwischen Vakuumphasen und Einleitung von Sterilgas entfernt. In einem letzten Schritt wird die Sterilisationskammer dann mit sterilem Gas geflutet, die Kammer kann geöffnet und das Sterilisiergut entnommen werden.

Um die Sterilität der Produkte auch für Transport und Lagerung zu gewährleisten, erfolgt die Sterilisation meist in beutelförmigen Umverpackungen. Diese bestehen auf einer Seite aus einer weitgehend gasundurchlässigen Sichtfolie und auf der anderen Seite aus einem gasdurchlässigen Papier, so dass das Sterilisationsmedium durch die Verpackung zu dem Sterilisationsgut gelangen kann

Die zu sterilisierenden Produkte, wie z. B. Schlauchsysteme werden unter Atmosphärendruck in den Umverpackungsbeutel eingelegt und dieser Beutel wird dann durch Verklebung oder Verschweißung dicht verschlossen. Ein Gasaustausch ist nur noch über das gasdurchlässige Papier möglich. Der Strömungswiderstand durch das Papier hindurch ist jedoch für den dargestellten Prozess signifikant und beeinflusst die Anzahl der durchzuführenden Bedampfungszyklen, um einen ausreichenden Austausch von Luft gegen Sterilisationsmedium zu erreichen.

Wird nun ein Vakuum an die Sterilisationskammer angelegt, entweicht zunächst das im Sterilisiergut befindliche Gas in den Umverpackungsbeutel. Der Beutel bläht sich auf das maximale Füllvolumen auf. Erst wenn dieses erreicht wird, erfolgt ein Gasaustausch durch den gasdurchlässigen Teil der Umverpackung. Bei dem Wechsel auf die Einströmphase wird der aufgeblähte Beutel komprimiert. Die darin befindliche Luft wird wieder in das Sterilisiergut zurückgedrückt. Durch das Hin- und Herpendeln der Luft zwischen Innenvolumen des Sterilisiergutes und Füllvolumen des Verpackungsbeutels, ist nur ein ineffizienter Austausch gegeben, weshalb vor der Inkubationsphase eine größere Anzahl an Evakuierungszyklen notwendig ist. Das gleiche gilt für die sich nach der Inkubation anschließenden Evakuierungszyklen zur Entfernung des Sterilisationsmediums.

Ein weiterer negativer Aspekt ist, dass sich der Verpackungsbeutel in vertikaler Richtung aufbläht, was in einer Verkleinerung des Querschnitts resultiert. Das kann abhängig von der Geometrie des Sterilisiergutes zu einem verstärkten Kontakt desselben mit der Verpackungsfolie führen, so dass bei den erhöhten Temperaturen, bei denen die Sterilisierung stattfindet, Verklebungen auftreten können.

Aufgabe der vorliegenden Erfindung ist es, ein verbessertes Sterilisationsverfahren zur Verfügung zu stellen. Ein solches Sterilisationsverfahren soll z.B. einen effizienteren Gasaustausch in der Umverpackung erreichen.

Weiterhin sollen Verklebungen zwischen der Sichtfolie der Umverpackung und dem Inhalt der Umverpackung vermieden werden.

Nach der Lehre der vorliegenden Erfindung wird diese Aufgabe gelöst durch ein Sterilisationsverfahren nach Anspruch 1, einer Sterilisationsvorrichtung nach Anspruch 10 und der Verwendung eines Füllkörpers nach Anspruch 15. Besondere Ausgestaltungen sind Gegenstand der Unteransprüche.

### Zusammenfassung der Erfindung

Bei dem erfindungsgemäßen Sterilisationsverfahren wird der zu sterilisierende Gegenstand oder werden die zu sterilisierenden Gegenstände, in der Folge Sterilisationsgut genannt, in eine evakuierbare Sterilisationskammer eingelegt. Dann wird ein Füllkörper eingelegt. Die Reihenfolge ist hierbei ohne Belang, der Füllkörper kann auch zuerst eingelegt werden, beide können auch gleichzeitig in die Sterilisationskammer eingebracht werden. Anschließend wird die Sterilisationskammer evakuiert, wobei vorzugsweise ein Druck kleiner 110 mbar eingestellt wird. Ist der gewünschte Unterdruck erreicht, wird ein Sterilisationsmedium in die Sterilisationskammer geleitet. Dadurch steigt der Druck in der Sterilisationskammer wieder an. Vorzugsweise kann ein Druck größer 2 bar eingestellt werden. Die Evakuierung der Sterilisationskammer und das Einleiten des Sterilisationsmediums wird so oft wiederholt, bis die zu Anfang in der Sterilisationskammer und in den zu sterilisierenden Gegenständen vorhandene Luft durch eine nahezu gesättigte Sterilisationsmediumsatmosphäre ausgetauscht ist. Nahezu bedeutet hier, dass der Sättigungsgrad ausreicht um eine Sterilisierung zu gewährleisten, auch eine gesättigte Sterilisationsmediumsatmosphäre soll davon umfasst sein. Dann erfolgt eine Inkubation mit dem Sterilisationsmedium, d. h. das Sterilisationsmedium wird für einen vorbestimmten Zeitraum in der Sterilisationskammer belassen, bis ein vorbestimmtes Niveau an Entkeimung erzielt ist.

Anschließend wird die Sterilisationskammer wieder evakuiert und dann mit einem Sterilgas, z.B. steriler Luft, geflutet.

Diese beiden Schritte werden so oft wiederholt, bis das Sterilisationsmedium aus der Sterilisationskammer und dem Sterilisationsgut entfernt ist. Dann wird die Sterilisationskammer geöffnet und das Sterilisationsgut aus der Kammer entnommen.

Bei dem erfindungsgemäßen Verfahren können alle gängigen, gasförmigen Sterilisationsmedien, wie z.B. Heißdampf, Wasserstoffperoxid, Ozon oder EtO eingesetzt werden.

Der Austausch der Luft durch das Sterilisationsmedium kann durch die Verwendung eines Füllkörpers beschleunigt werden, da dieser durch sein Volumen einen Teil der Umgebungsluft in der Sterilisationskammer ersetzt. Bei dem Füllkörper handelt es sich um einen flexiblen, geschlossenen, gashaltigen Hohlkörper. Es kann sich vorzugsweise um einen geschlossenen Kunststoffbeutel, z.B. ein Kunststoffbeutel, der an allen vier Seiten verschweißt ist, handeln, der eine geringe Gasmenge ausweist, der aber nicht vollständig gefüllt ist. Entsteht nun durch Abpumpen der Luft in der Sterilisationskammer ein Unterdruck, so dehnt sich der Beutel auf sein maximal zu erreichendes Volumen aus. Der Raum, der zu evakuieren ist, wird damit verkleinert, der Evakuationsschritt kann beschleunigt werden.

Ein zusätzlicher positiver Effekt kann bei verpacktem Sterilisationsgut erreicht werden. In einer bevorzugten Ausführungsform kann sich das Sterilisationsgut in einer gaspermeablen Umverpackung befinden. Die gaspermeable Umverpackung kann auf einer Seite aus einer gaspermeablen Schicht, z.B. einer Papierschicht, und auf einer zweiten Seite aus einer weitgehend gasdichten Sichtfolie bestehen.

Die gaspermeable Umverpackung kann in einer bevorzugten Ausführungsform flexibel sein. Durch die gaspermeable Verpackung kann im Evakuierungsschritt Luft aus der Verpackung austreten, umgekehrt kann bei Beladung der Sterilisationskammer mit Sterilisationsmedium dasselbe in die Verpackung eindringen.

Bei den bekannten Vakuumsterilisationsverfahren dehnt sich der Umverpackungsbeutel, der bei Umgebungsdruck befüllt und verschlossen wurde, während des Evakuierungsschritts durch die Drucksenkung zunächst bis zu seinem maximalen Volumen. Erst dann erfolgt ein Durchtritt der in der Verpackung und dem Sterilisationsgut vorhandenen Luft durch die Verpackung in die Sterilisationskammer. Am Ende des Evakuierungsschritts verbleibt eine dem maximalen Volumen der Verpackung entsprechende Luftmenge in der Verpackung. Diese wird im nächsten Schritt, nämlich dem Einleiten des Sterilisationsmediums und der damit einhergehenden Druckerhöhung komprimiert und wieder in das Sterilisationsgut, das Hohlräume aufweisen kann, zurückgeschoben. Mit steigendem Druck wird dann auch Sterilisationsmedium in der Verpackung in die Hohlräume des Sterilisationsgutes transferiert. Durch den Füllkörper in der Sterilisationskammer wird nun der Raum für die Ausdehnung des Umverpackungsbeutels bei dem Evakuierungsschritt vermindert. Die Umverpackung kann sich nicht mehr bis zu dem maximalen Volumen ausdehnen. Damit erfolgt früher ein Transfer von Luft aus der Verpackung in die Sterilisationskammer. Da die Verpackung komprimiert ist, ist am Ende des Evakuierungsschrittes in der Verpackung weniger Luft vorhanden, die in das Sterilisationsgut zurückgeschoben werden kann. Dabei erweist sich ein flexibler Füllkörper als besonders vorteilhaft, weil sich dieser der Form des Gegenstands anpassen und so eine möglichst effiziente Raumrestriktion bilden kann. So kann ein flexibler Beutel in die Mulden der Verpackung eines Schlauchsystems eindrücken.

Der Füllkörper ist so ausgebildet, dass er sich erst im Evakuierungsschritt ausdehnt. Unter Umgebungsbedingungen ist er deutlich kleiner und kann so ohne Probleme zusammen mit dem Sterilisiergut in die Sterilisationskammer eingelegt und auch wieder entnommen werden.

Die Einsätze, z.B. Einlegeböden oder Einlegekörbe, zur Aufnahme des Sterilisationsgutes in der Sterilisationskammer sollten so angeordnet werden, dass beim Evakuierungsschritt durch die Ausdehnung des Füllkörpers die Umverpackung mit dem Sterilisationsgut zusammengedrückt und die Ausdehnung der Umverpackung gering gehalten wird.

Der Füllkörper sollte vorzugsweise in seiner Grundfläche im Wesentlichen mindestens so groß sein, wie die Umverpackung. In einer besonders bevorzugten Ausführungsform entspricht die Grundfläche des Füllkörpers in etwa der Grundfläche der Einsätze in der Sterilisationskammer, die zur Aufnahme des Sterilisationsgutes dienen.

In einer besonders bevorzugten Ausführungsform wird zuerst ein Umverpackungsbeutel in die Sterilisationskammer eingelegt, dann ein Füllkörper und darauf wiederum ein Umverpackungsbeutel, so dass sich der Füllkörper zwischen den beiden Umverpackungsbeuteln befindet. Der zwischen den beiden Umverpackungsbeuteln angeordnete Füllkörper kann dann während des Evakuierungsschrittes die Ausdehnung von zwei Umverpackungsbeuteln verhindern.

Das erfindungsgemäße Verfahren kann vorzugsweise bei der Sterilisation von Gegenständen angewendet werden, die luftgefüllte, zum Umgebung offene Hohlräume aufweisen, besonders bevorzugt sind dabei Schlauch- oder Kassettensysteme.

Bei dem erfindungsgemäßen Verfahren können alle gängigen Sterilisationsvorrichtungen verwendet werden, bei denen in der Sterilisationskammer ein Vakuum bzw. Unterdruck erzeugt werden kann.

Die Erfindung betrifft weiterhin eine Sterilisationsvorrichtung mit einer evakuierbaren Sterilisationskammer, einem Mittel zur Erzeugung eines Vakuums und einem Mittel zur Bereitstellung eines Sterilisationsmediums.

Die Sterilisationsvorrichtung ist dadurch gekennzeichnet, dass die Sterilisationskammer einen Füllkörper aufweist.

Bei dem Füllkörper handelt es sich um einen flexiblen, gashaltigen, geschlossenen Hohlkörper, z.B. um einen geschlossenen Kunststoffbeutel handeln.

Die Sterilisationsvorrichtung kann weiterhin ein Mittel zur Erzeugung eines Sterilisationsmediums aufweisen, so kann dieses Mittel z.B. Ozon erzeugen.

Die Sterilisationsvorrichtung kann weiterhin ein Mittel zur Zufuhr des Sterilisationsmediums in die Sterilisationskammer aufweisen. Dieses Mittel kann identisch sein mit dem Mittel zur Erzeugung des Vakuums.

Bei dem Mittel zur Erzeugung kann es sich um eine gängige Vakuumpumpe handeln, z.B. eine Drehschieberpumpe oder eine Membranpumpe.

Darüber hinaus kann die Sterilisationsvorrichtung eine Steuer- oder Regeleinheit aufweisen, wodurch die Durchführung des erfindungsgemäßen Verfahrens steuerbar und/oder regelbar und/oder überwachbar wird, vorzugsweise halbautomatisch und/oder vollautomatisch steuerbar und/oder regelbar und/oder überwachbar.

Ein weiterer Aspekt der Erfindung ist die Verwendung eines Füllkörpers, eines flexiblen, gasgefüllten Kunststoffbeutels, in einer Sterilisationsvorrichtung wie oben beschrieben oder in einem Verfahren der fraktionierten Vakuumsterilisation.

In den Figuren werden beispielhafte Ausführungsformen der Erfindung dargestellt.

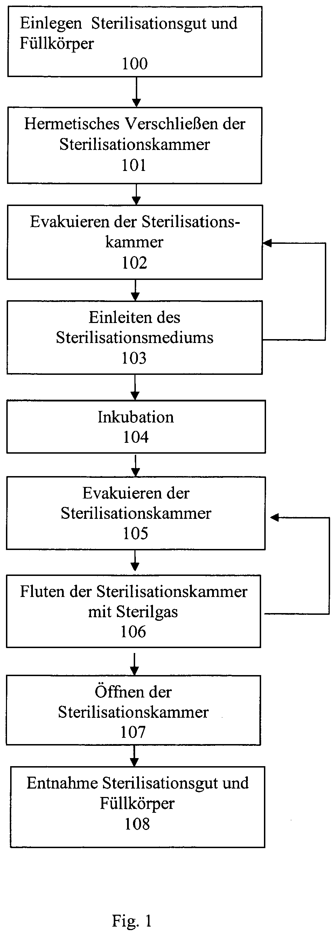

Figur 1 zeigt den Verfahrensablauf in Form eines Flussdiagramms.
Figur 2 zeigt die schematische Ansicht einer beispielhaften Sterilisationsvorrichtung.
Figur 3 zeigt schematisch die Sterilisationskammer einer Sterilisationsvorrichtung nach dem Stand der Technik mit Sterilisationsgut in einer Umverpackung in belüftetem Zustand (A) und evakuiertem Zustand (B).
Figur 4 zeigt schematisch eine Ausführungsform einer Sterilisationskammer einer erfindungsgemäßen Sterilisationsvorrichtung mit Sterilisationsgut in einer Umverpackung in belüftetem Zustand (A) und evakuiertem Zustand (B).

### Beschreibung

Bei dem erfindungsgemäßen Verfahren wird im ersten Schritt 100 Sterilisationsgut und Füllkörper in die Sterilisationskammer eingebracht. Die Sterilisationskammer wird dann hermetisch in einem Schritt 101 verschlossen und dann in einem Schritt 102 evakuiert. Ist der gewünschte Unterdruck erreicht, folgt das Einleiten der Sterilisationsmediums 103. Die bis in der Sterilisationskammer eine nahezu gesättigte Atmosphäre des der Sterilisationsmediums erreicht ist. Dann erfolgt eine Inkubation 104 des Sterilisationsguts, bis der gewünschte Grad an Entkeimung erreicht ist. Die Sterilisationskammer wird dann in einem Schritt 105 wieder evakuiert und anschließend mit einem sterilen Gas in einem Schritt 106 belüftet. Die Schritte 105 und 106 werden so oft wiederholt, bis das Sterilisationsmedium nahezu vollständig aus der Sterilisationskammer entfernt ist. Nach einer letzten Belüftung 106 kann die Sterilisationskammer geöffnet und das Sterilisationsgut entnommen werden.

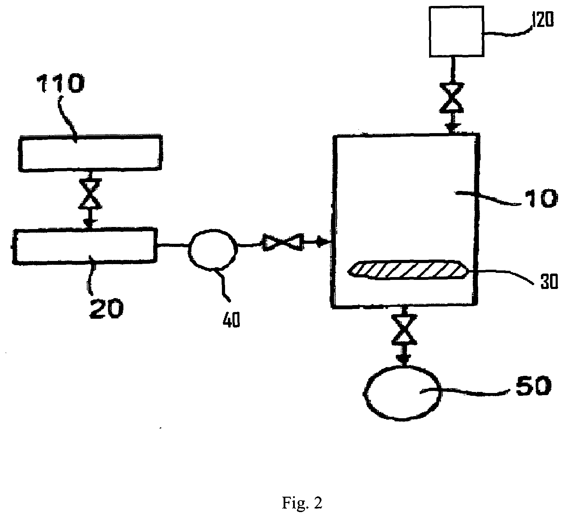

In Figur 2 ist eine erfindungsgemäße Vorrichtung zur Sterilisation dargestellt. In der Sterilisationskammer 10 befindet sich ein Füllkörper 30, dessen Volumen sich während der Evakuierungsschritte 102 und 105 stark ausdehnen kann. Die Evakuierung erfolgt über eine Vakuumpumpe 50, die an die Sterilisationskammer angeschlossen ist. Weiterhin ist an die Sterilisationskammer 10 ein Mittel zur Bereitstellung eines Sterilisationsmediums 20 angeschlossen. Das Sterilisationsmedium kann aus diesem Gefäß 20 über eine Pumpe 40 in die Sterilisationskammer eingeführt werden, das Sterilisationsmedium kann aber auch durch die Vakuumpumpe 50 in die Sterilisationskammer überführt werden. In das Gefäß 20 kann das Sterilisationsmedium aus einem Mittel zur Erzeugung dieses Sterilisationsmediums 110 zugeführt werden. Dabei kann es sich z.B. um eine Dampferzeugung oder eine Ozonerzeugung handeln. Weiterhin weist die Sterilisationskammer 10 eine Leitung zu einer Quelle für ein Sterilgas 120 auf.

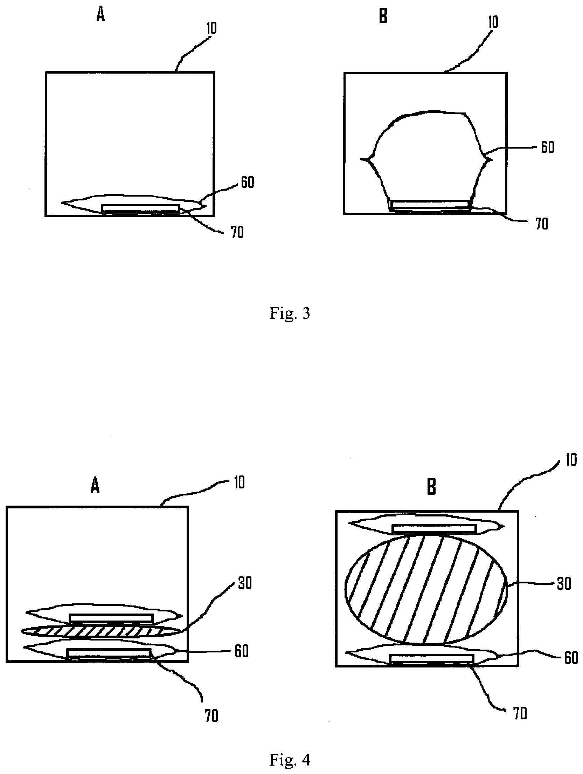

In Figur 3 ist eine Sterilisationskammer 10 mit Sterilisationsgut 70 nach dem Stand der Technik gezeigt. Das Sterilisationsgut 70 liegt in einer Umverpackung vor. In Figur 3A ist die Sterilisationskammer 10 bei Umgebungsdruck oder in der Phase mit Beladung mit Sterilisationsmittel gezeigt. In Figur 3B ist die Sterilisationskammer 10 in der Evakuierungs- oder Vakuumphase gezeigt. Die Umverpackung hier ist bis auf das maximale Packungsvolumen aufgebläht.

In Figur 4 ist ein Ausschnitt aus einer erfindungsgemäßen Sterilisationsvorrichtung gezeigt. In der Sterilisationskammer 10 ist zwischen 2 Umverpackungen mit Sterilisationsgut 70 ein als Kunststoffbeutel ausgebildeter Füllkörper 30 angeordnet. In Figur 4A ist die Sterilisationskammer 10 bei Umgebungsdruck oder in der Phase mit Beladung mit Sterilisationsmittel gezeigt. In Figur 4B ist die Sterilisationskammer 10 in der Evakuierungs- oder Vakuumphase gezeigt. Im Unterschied zum Stand der Technik hat sich hier der Kunststoffbeutel 30 bis zu seinem Maximalvolumen aufgebläht. Die Dimensionen in der Sterilisationskammer 10 sind so gewählt, dass durch die Raumforderung des Kunststoffbeutels 30 in der Evakuierungsphase eine Ausdehnung der Umverpackung um das Sterilisiergut vermindert wird. Im Unterschied zum Kunststoffbeutel 30 ist die Umverpackung gaspermeabel, so dass durch den Kunststoffbeutel 30 die Luft aus der Verpackung herausgedrückt wird. Da die Raumforderung des Kunststoffbeutels bei Umgebungsdruck deutlich kleiner ist, können Kunststoffbeutel und Sterilisationsgut einfach in die Sterilisationskammer eingelegt und entnommen werden.

Durch das erfindungsgemäße Verfahren und die erfindungsgemäße Vorrichtung kann die Anzahl der notwendigen Vakuum-Druck-Zyklen vermindert werden. Damit kann eine Verkürzung der Sterilisationszeit, eine Einsparung an Sterilisationsmedium und an Energie erreicht werden.

## Patentansprüche

1. Verfahren zum Sterilisieren von Sterilisationsgut in einer evakuierbaren Sterilisationskammer umfassend folgende Verfahrensschritte:
a) Einbringen des Sterilisationsguts und mindestens eines Füllkörpers in die evakuierbare Sterilisationskammer
b) Hermetisches Verschließen der Sterilisationskammer
c) Evakuieren der Sterilisationskammer und damit einhergehende Senkung des Drucks
d) Einleiten eines Sterilisationsmediums in die Sterilisationskammer und damit einhergehende Anhebung des Drucks
e) Wiederholen der Schritte c) bis d) nach Bedarf um in der Sterilisationskammer und dem Sterilisationsgut eine nahezu gesättigte SterilisationsmediumsAtmosphäre zu erzielen
f) Belassen des Sterilisationsmediums in der Sterilisationskammer für einen vorbestimmten Zeitraum um ein vorbestimmtes Niveau an Entkeimung zu erzielen
g) Evakuieren der Sterilisationskammer
h) Fluten der Sterilisationskammer mit einem Sterilgas
i) Wiederholen der Schritte g) bis h) nach Bedarf um das Sterilisationsmedium aus der Sterilisationskammer und dem Sterilisationsgut zu entfernen
j) Öffnen der Sterilisationskammer
k) Entnehmen des Sterilisationsguts und des Füllkörpers aus der Sterilisationskammer
**dadurch gekennzeichnet, dass** es sich bei dem Füllkörper um einen flexiblen, geschlossenen, gashaltigen Hohlkörper handelt.

2. Verfahren nach Anspruch 1, **dadurch gekennzeichnet, dass** bei Schritt c) der Druck auf kleiner 110mbar abs. abgesenkt wird.

3. Verfahren nach Anspruch 1, **dadurch gekennzeichnet, dass** bei Schritt d) der Druck auf größer 2bar abs. angehoben wird.

4. Verfahren nach Anspruch 1, **dadurch gekennzeichnet, dass** es sich bei dem Hohlkörper um einen Kunststoffbeutel handelt.

5. Verfahren nach einem der vorangehenden Ansprüche, **dadurch gekennzeichnet, dass** sich das Sterilisationsgut in einer gaspermeablen Umverpackung befindet.

6. Verfahren nach einem der vorangehenden Ansprüche, **dadurch gekennzeichnet, dass** die gaspermeable Umverpackung flexibel ist.

7. Verfahren nach einem der vorangehenden Ansprüche, **dadurch gekennzeichnet, dass** das Sterilisationsgut luftgefüllte, zur Umgebung offene Hohlräume aufweist.

8. Verfahren nach Anspruch 5, **dadurch gekennzeichnet, dass** es sich bei dem Sterilisationsgut um Schlauchsysteme handelt.

9. Verfahren nach Anspruch 5, **dadurch gekennzeichnet, dass** es sich bei dem Sterilisationsgut um Kassettensysteme handelt.

10. Sterilisationsvorrichtung zur Durchführung des Verfahrens nach Anspruch 1 umfassend eine evakuierbare Sterilisationskammer (10), ein Mittel zur Erzeugung eines Vakuums (50) und ein Mittel zur Bereitstellung eines Sterilisationsmediums (20) **dadurch gekennzeichnet, dass** die Sterilisationskammer mindestens einen Füllkörper (30) in Form eines geschlossenen, gashaltigen Hohlkörpers aufweist.

11. Vorrichtung nach Anspruch 10 **dadurch gekennzeichnet, dass** es sich bei den Füllkörpern um einen Kunststoffbeutel handelt.

12. Vorrichtung nach Anspruch 11 oder 10 **dadurch gekennzeichnet, dass** sie ein Mittel zur Zufuhr des Sterilisationsmediums in die Sterilisationskammer und ein Mittel zur Bereitstellung eines Sterilisationsmediums aufweist.

13. Vorrichtung nach Anspruch 10, 11 oder 12 **dadurch gekennzeichnet, dass** sie eine Steuer- oder Regelungseinheit zur Durchführung des Verfahrens nach Anspruch 1 bis 9 aufweist.

14. Verwendung eines Füllkörpers in einem Verfahren nach Anspruch 1 bis 9 oder einer Vorrichtung nach Ansprüchen 10 bis 13.

## Claims

1. A method for sterilizing items for sterilization in a sterilization chamber that can be evacuated, comprising the following method steps:
a) introducing the items for sterilization and at least one packing into the evacuable sterilization chamber;
b) hermetically sealing the sterilization chamber;
c) evacuating the sterilization chamber and lowering the pressure accordingly;
d) introducing a sterilization medium into the sterilization chamber and raising the pressure accordingly;
e) repeating steps C) through d) as needed to achieve an almost saturated sterilization medium atmosphere in the sterilization chamber and the items for sterilization;
f) leaving the sterilization medium in the sterilization chamber for a predetermined period of time to achieve a predetermined level of disinfection;
g) evacuating the sterilization chamber;
h) flooding the sterilization chamber with a sterile gas;
i) repeating steps g) through h) as needed to remove the sterilization medium from the sterilization chamber and the items for sterilization;
j) opening the sterilization chamber;
k) removing the items for sterilization and the packing from the sterilization chamber;
**characterized in that** the packing is a closed, flexible, hollow body containing a gas.

2. The method according to claim 1,
**characterized in that** the pressure is lowered to less than 110 mbar abs. in step c).

3. The method according to claim 1,
**characterized in that** the pressure is raised to more than 2 bar abs. in step d).

4. The method according to claim 1,
**characterized in that** the hollow body is a plastic bag.

5. The method according to any one of the preceding claims, **characterized in that** the items for sterilization are in a gas-permeable outer package.

6. The method according to any one of the preceding claims, **characterized in that** the gas-permeable outer package is flexible.

7. The method according to any one of the preceding claims, **characterized in that** the items for sterilization has air-filled cavities that are open to the .

8. The method according to claim 5,
**characterized in that** the items for sterilization are tubing systems.

9. The method according to claim 5,
**characterized in that** the items for sterilization are cassette systems.

10. A sterilization device for carrying out the method according to claim 1, comprising an evacuable sterilization chamber (10), a means for creating a vacuum (50) and a means for supplying a sterilization medium (20),
**characterized in that** the sterilization chamber has at least one packing (30) in the form of a closed hollow body containing gas.

11. The device according to claim 10,
**characterized in that** the packing is a plastic bag.

12. The device according to claim 11 or 10,
**characterized in that** it has a means for supplying the sterilization medium to the sterilization chamber and a means for supplying a sterilization medium.

13. The device according to claim 10, 11 or 12,
**characterized in that** it has a control or regulating unit for carrying out the method according to claim 1 to claim 9.

14. Use of a packing in a method according to claims 1 to 9 or a device according to claims 10 to 13.

## Revendications

1. Procédé de stérilisation de produits à stériliser dans une chambre de stérilisation pouvant être mise sous vide, comprenant les étapes opératoires suivantes :
a) introduction du produit à stériliser et d'au moins un corps de remplissage dans la chambre de stérilisation pouvant être mise sous vide,
b) fermeture hermétique de la chambre de stérilisation,
c) mise sous vide de la chambre de stérilisation et baisse corrélative de la pression,
d) introduction d'un fluide de stérilisation dans la chambre de stérilisation et augmentation corrélative de la pression,
e) répétition des étapes c) à d) en fonction des besoins pour obtenir dans la chambre de stérilisation est le produit à stériliser une atmosphère pratiquement saturée de fluide de stérilisation,
f) le fait de laisser le fluide de stérilisation dans la chambre de stérilisation pendant un temps prédéfini pour obtenir un niveau prédéfini de désinfection,
g) mise sous vide de la chambre de stérilisation,
h) irrigation de la chambre de stérilisation avec un gaz stérile,
i) répétition des étapes g) à h) en fonction des besoins pour éliminer le fluide de stérilisation dans la chambre de stérilisation et le produit à stériliser,
j) ouverture de la chambre de stérilisation,
k) retrait du produit à stériliser et du corps de remplissage de la chambre de stérilisation,
**caractérisé en ce que** le corps de remplissage est un corps creux fermé contenant du gaz.

2. Procédé selon la revendication 1, **caractérisé en ce que**, dans l'étape c), la pression est descendue à moins de 110 mbar abs.

3. Procédé selon la revendication 1, **caractérisé en ce que**, dans l'étape d), la pression est montée à plus de 2 bar abs.

4. Procédé selon la revendication 1, **caractérisé en ce que** le corps creux et un sachet en plastique.

5. Procédé selon une des revendications précédentes, **caractérisé en ce que** le produit à stériliser se trouve dans un emballage perméable aux gaz.

6. Procédé selon une des revendications précédentes, **caractérisé en ce que** l'emballage perméable aux gaz est souple.

7. Procédé selon une des revendications précédentes,
**caractérisé en ce que** le produit à stériliser contient des qualités remplies d'air est ouverte vers l'environnement.

8. Procédé selon la revendication 5, **caractérisé en ce que** le produit à stériliser et un système de tuyaux.

9. Procédé selon la revendication 5, **caractérisé en ce que** le produit à stériliser et un système de cassettes.

10. Dispositif de stérilisation pour la réalisation du procédé selon la revendication 1, comprenant une chambre de stérilisation pouvant être mise sous vide (10), un moyen de génération d'un vide (50) est un moyen de mise à disposition d'un fluide de stérilisation (20),
**caractérisé en ce que** la chambre de stérilisation présente au moins un corps de remplissage (30) sous la forme d'un corps creux fermé contenant du gaz.

11. Dispositif selon la revendication 10, **caractérisé en ce que** le corps de remplissage et un sachet en plastique.

12. Dispositif selon la revendication 11 ou 10,
**caractérisé en ce qu'**il présente un moyen d'acheminement du fluide de stérilisation dans la chambre de stérilisation est un moyen de mise à disposition d'un fluide de stérilisation.

13. Dispositif selon la revendication 10, 11 ou 12,
**caractérisé en ce qu'**il présente une unité de commande ou de réglage pour la réalisation du procédé selon les revendications 1 à 9.

14. Utilisation d'un corps de remplissage dans un procédé selon la revendication 1 à 9 ou un dispositif selon les revendications 10 à 13.
